# EUROPEAN PATENT SPECIFICATION

(11) **EP 3 286 559 B1**
(45) Date of publication and mention of the grant of the patent: **04.09.2019**
(21) Application number: 15728154.4
(22) Date of filing: 20.04.2015
(51) Int. Cl.: G01N 29/02, G01N 33/18

(54) **THE USE OF PIEZOELECTRIC TRANSDUCERS MODIFIED WITH METAL OXIDE-BASED THIN FILMS FOR DIRECT DETECTION OF AMINE DERIVATIVES IN LIQUID MEDIA**
VERWENDUNG VON PIEZOELEKTRISCHEN WANDLERN, DIE MIT METALLOXIDBASIERTEN DÜNNSCHICHTEN MODIFIZIERT SIND, ZUR DIREKTEN DETEKTION VON AMINDERIVATEN IN FLÜSSIGEN MEDIEN
UTILISATION DE TRANSDUCTEURS PIÉZO-ÉLECTRIQUES MODIFIÉS AVEC FILMS MINCES À BASE D'OXYDE DE MÉTAL POUR LA DÉTECTION DIRECTE DE DÉRIVÉS D'AMINE DANS DES MILIEUX LIQUIDES

(43) Date of publication of application: 28.02.2018
(73) Proprietor: Tubitak, 06100 Ankara (TR)
(72) Inventor: ERBAHAR, Dilek, 41470 Kocaeli (TR); HARBECK, Mika, 41470 Kocaeli (TR); SEN, Zafer, 41470 Kocaeli (TR); KÖSEMEN, Arif, 41400 Kocaeli (TR); ÖZTÜRK, Sadullah, 41400 Kocaeli (TR); KILINÇ, Necmettin, 41400 Kocaeli (TR); ÖZTÜRK, Zafer Ziya, 41400 Kocaeli (TR); YERL , Yusuf, 41400 Kocaeli (TR)
(86) International application number: PCT/IB2015/052866
(87) International publication number: WO 2016/170385

(56) References cited:
- CN-B- 101 915 711
- US-A1- 2009 084 162
- US-A1- 2013 017 567
- LEE D ET AL: "Enhanced mass sensitivity of ZnO nanorod-grown quartz crystal microbalances", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, CH, vol. 135, no. 2, 15 January 2009 (2009-01-15), pages 444-448, XP025949842, ISSN: 0925-4005, DOI: 10.1016/J.SNB.2008.10.026 [retrieved on 2008-11-06]

## Description

### Technical Field Related to the Invention

This invention describes a method of detecting amine and amine derivatives in liquid media with high sensitivity and selectivity directly in the liquid phase. The use of this method allows a more efficient and easier detection of amines and amine derivatives in liquid media compared to existing techniques.

The method is based on the principle of detecting amines and amine derivatives in a liquid using vanadium pentoxide (V₂O₅) thin film coated electro-mechanical resonators such as the quartz crystal microbalance (QCM) operating directly in the liquid medium. Chemical sensors based on a QCM transducer coated with vanadium pentoxide (V₂O₅) as the sensitive material are used to detect amines and amine derivatives in liquid media in a fast, easy and accurate way. QCM transducers are mass-sensitive devices that can be used in gaseous and liquid media. They are durable, easy-to-use, economical, have high resolution, and allow real-time and in-situ measurements. QCMs are made of piezoelectric materials. Mass changes on the surface of the piezoelectric crystal allow detection with nanogram sensitivity by causing a change in the resonance frequency of the crystal. To detect analytes directly in a liquid medium, polymers and/or organic compounds are usually used as the sensitive material on the QCM-based transducer. However, metal oxides have not been used as sensitive materials on such chemical sensor systems working directly in a liquid medium. For analyte molecules contained in a liquid, the detection of the analytes using metal oxide sensors is described only by indirect means, e.g., by taking measurements in the gas phase above the liquid phase. Yet, owing to the method proposed here, analyte molecules contained in a liquid medium can be detected directly in the liquid phase without the need of any evaporation processes. In this work, a sensor is described for detecting amines and amine derivatives directly in the liquid medium with high sensitivity and selectivity using a metal-oxide material. Vanadium pentoxide (V₂O₅) was coated on a QCM transducer as a thin film using thermal evaporation. This coating method is quite advantageous since it minimizes surface roughness and enables an exact control of the coating thickness. The developed sensor was operated successfully in a liquid medium and showed high sensitivity and selectivity for amine derivatives.

The invention has also other advantages compared to exiting methods. For environmental analysis (identification of water pollution) analytical methods like gas chromatography (GC), high performance liquid chromatography (HPLC), capillary electrophoresis (CZE) and UV-VIS spectrophotometry are highly preferred. Although these methods have superior features in terms of sensitivity and selectivity, they are chemical analysis methods that are costly, require expert personal to operate, and yield results only after an intense effort and long analysis times. Besides, the chromatographic methods are due to their size and power and supply gas/carrier liquid requirements mostly used in fixed installations in a laboratory environment. Thus, for analysis of field samples, samples have to be transported to the laboratory causing problems with sample ageing and making continuous monitoring over long periods difficult. Furthermore, if derivatization of analyte molecules prior to analysis is necessary for sensitive detection, an additional source of error is introduced into the analysis procedure. Unlike the methods mentioned above that are used to determine and monitor the level of environmental pollution, in-situ and or at-site measurements can be easily done with the developed sensor. Besides, measurements taken with the developed sensor are less time consuming and more economical.

The new sensor is able to detect amines and amine derivatives quickly and in water with a sensitivity that passes lower detection limits set by environmental protection agencies [e.g. the US Environmental Protection Agency (EPA)]. This sensor can detect amines and amine derivatives in real-time directly in the liquid medium without the need of any evaporation or derivatization processes.

Patent document US-A-2009/0084162 discloses a chemical gas sensor comprising a piezoelectric substrate and a layer of vanadium pentoxide as sensitive layer.

### Technical Problems That the Invention Aims To Solve

Today, industrial facilities including paint, medical, cosmetics and plastic industries, or coal conversion facilities release high amounts of amines and amine derivatives into the environment. Besides, there is an amine release to the environment as a result of degradation of amino acids and proteins that are the elements of biological systems (vegetable or animal decomposition). Amines and amine derivatives released to the environment cause environmental pollution and, thus, become a factor that threatens human health. For example, aniline and its derivatives are quite dangerous due to their combined toxic as well as carcinogenic effects. Amines can turn into mostly carcinogenic "N-nitroso" compounds in a reaction with nitrosylation agents. Chlorinated amine compounds such as the high carcinogenic p-chloroaniline and 3,4 dichloroaniline can emerge as a degradation or intermediate product of pesticides such as phenylurea and phenylcarcarbamate. Many aliphatic amines themselves are toxic or highly biologically active. Besides, aliphatic amines are known to yield carcinogen products by readily undergoing reactions with nitrogen containing compounds. As such amines and amine derivatives are present only in small amounts in water, a measurement system to be used for reliable detection and classification needs to have high sensitivity and selectivity.

Methods such as gas chromatography (GC), high performance liquid chromatography (HPLC), capillary electrophoresis (CZE), and UV-VIS spectrophotometry are commonly used in the analyses of water pollution. These methods are usually employed due to their high sensitivity and selectivity in environmental analyses. However, these methods are costly, laborious, time consuming, and require a trained operator. Therefore, fast, easy-to-use and cost-effective detection systems are needed to determine and to track the level of water pollution.

In summary, low-cost, fast, and reliable detection systems are needed nowadays for detecting amine and amine derivatives in liquid media that are serious threats to the environment and to human health due to their high carcinogenic and toxic effects. This invention describes an effective measurement method to detect amines and amine derivatives in liquid media with high sensitivity and quick responses compared to existing techniques.

### Background of the invention

For the detection of carcinogenic or toxic amines, which humans can come in contact with in natural or artificial waters (lakes, rivers, or open seas, swimming pools, drinking water reservoirs), there is a demand of analytical instrumentation with high sensitivity, being portable, and allowing fast and reliable on-site detection.

The main amine emission sources to the environment are industrial facilities such as petroleum refining plants, production plants of synthetic polymers, paints, tires, pharmaceuticals, and explosives. Azo dyes, exhaust gases, protein degradation processes or decomposition of rich plants (forest fires), as well as meat consumption are intensively under research as non-industrial sources of amine compounds and their risk to cause cancer [1, 2]. Amines are considered as priority pollutants in the US Environmental Protection Agency (EPA) list [3]. The known methods for identifying amines and amine derivatives (aromatic amines, aliphatic amines) in liquid media are gas chromatography (GC), high performance liquid chromatography (HPLC), photometry, ion mobility spectrometry (IMS) and capillary electrophoresis (CZE) [1, 4]. Nowadays, the research on amine sensors that are highly sensitive and easy to use in liquid media is being continued because the classical analysis methods are used mainly in the laboratory and required an expert operator. In analytical chemistry, the development of chemical sensor systems for the detection and identification of organic compounds still poses a major challenge. For selective detection of each target compound sensors having different sensitive structures as sensitive element have to be developed. These sensors often need to be re-designed because of a low sensor performance in liquid media. However, Suslick et al. showed the usage of pH indicators, solvatochromic dyes, and metal ion-containing dyes for detecting organic compounds in liquids [5]. In addition, the research on the detection of chemical contaminants directly in liquids using QCM-based transducer has gained an increasing interest.

It was shown by Sauerbrey in 1959 that the frequency shift of a QCM is proportional to the mass change on the quartz crystal [6]. According to this principle, it was proposed that quartz crystals coated with various sensitive materials can be used in a gas chromatograph as a new type of detector [7]. The frequency shift as the result of ad/absorption of gas molecules on/into the sensitive layer provides a quantitative information about the composition of the analyte gas. This type of a chemical detector based on gravimetry was later named the quartz crystal microbalance (QCM) and has become one of the most widely used types of chemical gas sensors. However, it was for long thought to be unsuitable for liquid media applications. But, nowadays QCM-based sensors have become a transducer platform for sensing applications in liquid media, as well, and the interest in such sensors is increasing each day [8]. The frequency changes of the first QCM sensors operating in liquids were determined only after drying the crystal [9]. The first experiments were carried out for the detection of cyanide ions in aqueous solution in 1980 and showed the potential of the QCM for applications in liquids [10]. In another study, QCM sensors modified with silane monolayers are described as a detector for liquid chromatography [11]. The increase in the interest in sensors working in liquid media, especially in biosensors, has led to the development of the first QCM immunosensor with a liquid flow system that allows fully automated measurements [12]. The establishment of theoretical models describing the phenomena related to a QCM working in a fluid has accelerated the progress of the experimental work. The relationship between the frequency shift of a quartz crystal in a liquid medium, fluid density and viscosity was first formulated by Kanazawa and Gordon in their study published in 1985 [13]. In 1991, Martin et al. derived the final version of the equation for the dependency between the frequency shift of the quartz crystal in a liquid and the properties of the liquid by combining the Kanazawa-Gordon and Sauerbrey equations [14]. Okahata et al. have pioneered in the development of chemical QCM sensors working in a fluid medium. Crystals coated with a synthetic lipid bilayer sensitive material were used to detect bitter substances such as strychnine [15]. Then, variations in the sensitive material coating have allowed the selective detection of various analytes [16]. Generally, regular polymer films [17-23], films of organic macromolecules such as calixarenes [24, 25], self-organized monolayer films [26], phthalocyanines [27-31] etc. have been used as sensitive materials for the detection of organic compounds in liquid media with the QCM by taking chemical / physical properties into account.

Recently, selectivity and sensitivity for the detection of aromatic hydrocarbons have been increased by polymers modified with nano materials such as carbon nanotubes [32] or doped with plasticizers [33]. Molecular imprinting is a systematic approach to increase the performance of QCM sensors and their selectivity for specific target analytes. First reported examples in this field include the selective detection of s-propranolol in acetonitrile solution [34], as well as glucose [35], polycyclic aromatic hydrocarbons [36] in water, or terpenes in ethanol [37].

Work on metal oxide materials using QCM technology that prove their operation as a chemical sensor directly in liquids has not been reported yet. Even the use of metal oxides on QCM gas sensors as sensitive layers is very limited in number. Such gas sensors were used for the detection of volatile organic compounds [38]. Recently, the sensing properties to toxic gases using nanostructured WO₃, TiO₂, and CuO functionalized QCM sensors have been reported [39]. CuO functionalized QCM resonators were fabricated and explored for HCN sensing [40,41]. WO₃ [42,43], TiO₂ and fluorine doped titanium dioxide (F-TiO₂) [44] have been described for dimethyl methylphosphonate detection. A zinc oxide based nano structure modified QCM was used as a biosensor for the dynamic and noninvasive monitoring of the adhesion and proliferation of cells through multimode operation (acoustic and optical) [45].

In general, metal oxides (ZnO, TiO₂, V₂O₅, WO₃, ...) materials are mostly used in gas sensors based on a change in conductivity [46]. For amine detection in the gas phase a conductivity-type transducer with V₂O₅ as sensitive materials was used and rather attractive result have been obtained. The sensitivity of the V₂O₅ for amines is proportional to the humidity level in the ambient as shown by Raible and co-workers [47,48]. However, the operating capability of a V₂O₅ or any other metal oxide based chemical sensor directly in the liquid phase has not been demonstrated. Only, the indirect sensing of amines found in a liquid phase by measuring in the headspace above the liquid was proposed using a metal oxide gas sensor [49].

V₂O₅ shows selective catalytic activity for nitrogen oxide compounds [50]. Besides, the diffusion of amine groups on V₂O₅ surface is accelerated by its hygroscopic properties and the amine sensitivity of V₂O₅ gas sensors is enhanced by ambient humidity [47,48,51]. These properties make V₂O₅ a promising, highly capable sensing material for the fast and selective detection of amines and amine groups containing compounds directly in the liquid sample medium, as well.

### Details of the invention

The invention is directed at the use of vanadium pentoxide as sensitive material on piezoelectric transducer based chemical sensors for fast, easy, and sensitive detection of amines and amine derivatives in water. The quartz crystal microbalance transducer is a mass sensitive device produced using piezoelectric materials and applicable in gas and liquid phase sensing applications, durable, easy to use, cost effective, allowing high resolution, real time, on site/in situ measurements. The mass change occurring on the piezoelectric crystal surface alternates the crystal's resonance frequency enabling detection on a nanogram level.

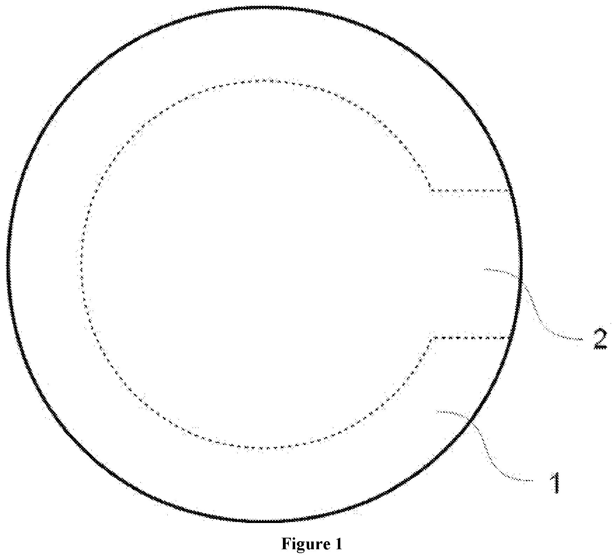

Vanadium pentoxide is used for the first time as sensitive material on chemical sensors working directly in liquids. Being sensitive to amines and amine derivatives the metal oxide V₂O₅ was coated on the active electrode (**2**) of the 5 MHz QCM transducers (**1**) by the heat evaporation method as a thin layer (**4**) of 20 nm thickness (see figures 1-3).

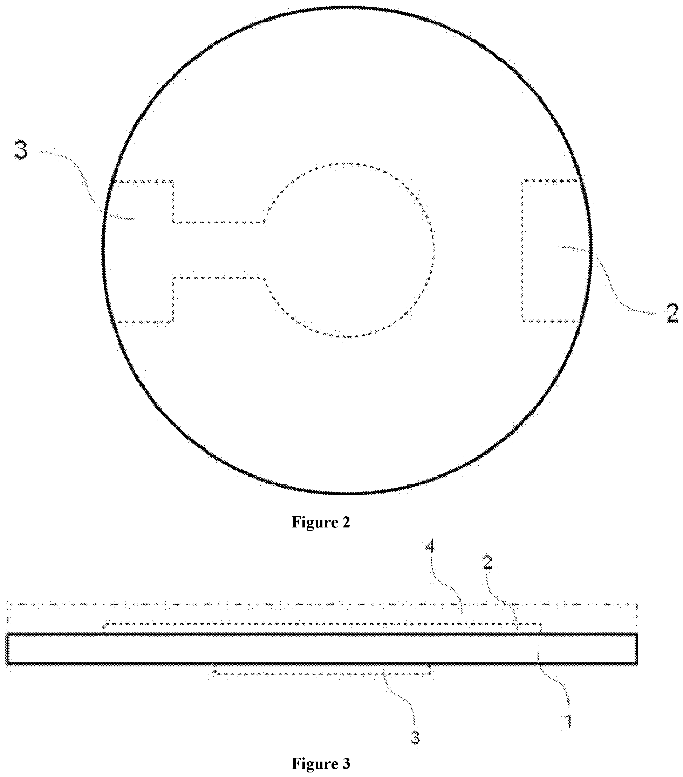

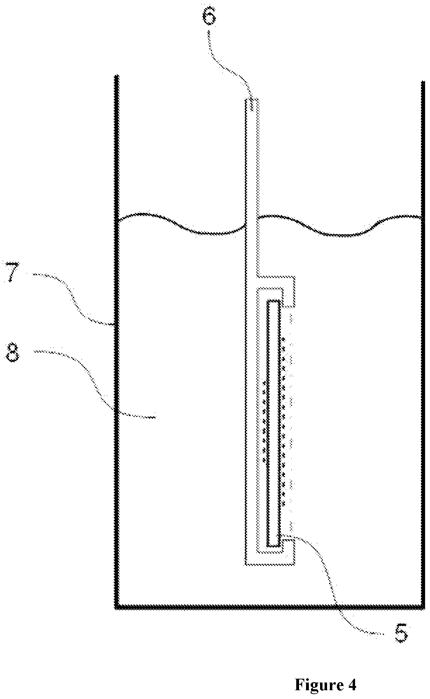

The analytes to be tested are added in pure form with the help of a micropipette or analytical scale to a given volume of pure water according to their water solubility. Then, the solution is mixed by shaking for a fixed time. Before each measurement a new stock solution was prepared. The necessary concentration for the measurement is produced by diluting the stock solution to the desired level. For the sensor tests, a KSV QCM-Z500 device (KSV Instruments, Finland) with a temperature stabilized measurement cell was used. With the KSV QCM-Z500 the resonance frequency in the 5-55 MHz frequency range and quality factor of the resonance (Q) of a quartz crystal immersed in water can be determined by impedance analysis. The temperature of the measurement cell was kept constant at 20°C during all measurements. The front side with the V₂O₅ thin film (4) and the active electrode (**2**) of the V₂O₅ coated QCM (**5**) was fully in contact with the liquid medium during the measurement (see sketch in figure 3,4). It was made sure that the samples containing the analytes and the carrier liquid (pure water) have been adjusted to the same temperature before reaching the measurement cell. The following procedure resembles a typical measurement protocol: the sensor is first exposed to the carrier liquid (pure water) to obtain the initial value of the sensor (baseline) and then to the analyte containing solution until a stable signal is maintained. At last, the sensor is purged again with the carrier liquid to reset the baseline. The sensors signal is recorded continuously over the whole measurement period. This cycle is repeated for all analyte and concentration levels. The sensor signal obtained during exposure to the carrier liquid is taken as the base (denoted as f₀). The sensor response as the frequency shift Δf is calculated from the difference of the resonance frequency during analyte exposure at equilibrium (fₛ) to the baseline (f₀). The observable change in frequency depends on the number and weight of the molecules bound to the QCM surface. Due to interactions between the sensitive material on the QCM with analyte molecules during exposure with the sample solution analyte molecules are bound to the QCM leading to a measureable frequency shift. During the measurement all other parameters influencing the QCM such as temperature and liquid density are kept constant.

The developed sensor was tested against selected amines (triethylamine, butylamine, hexylamine and chloramine T) as target analytes and volatile organic compounds (dichloromethane, chloroform, chlorbenzene, trichloroethylene, tetrachloroethylene, and p-xylene), phenols (bisphenol A), and various pesticides (methiocarb, propoxur, triadimenol, tebuconazole, iprodine, and triadimefon) as interferents potentially present in water.

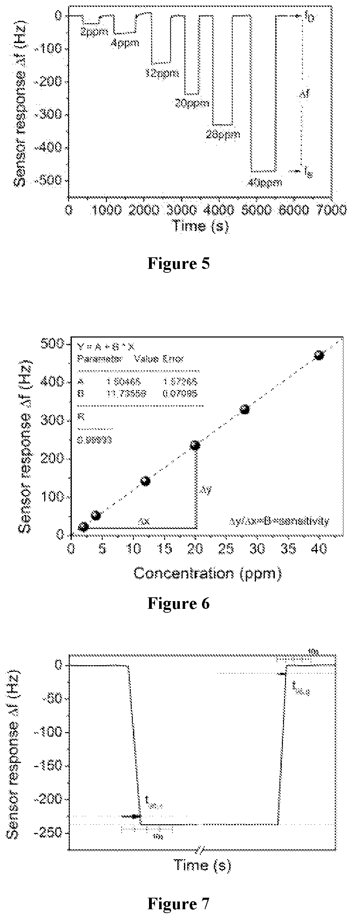

To evaluate the sensor performance in detail the negative frequency shifts upon exposure to different concentrations of the analytes, i.e. the sensor responses, were determined. In figure 5, the sensor responses of a V₂O₅ coated QCM sensor is shown during exposure to chloramine T dissolved in water at different concentration levels. The calculation of the sensor responses is illustrated using the sensor signal to the highest measured concentration level. The baseline QCM frequency is set to zero. The calibration curve is obtained from the linear regression of the sensor responses versus the different analyte concentrations. The sensor sensitivity is defined as the slope Δy/ Δx of the sensor response curve. In figure 6, the responses of the sensor as a function of analyte concentration in the sample are plotted together with the best-fit linear regression curve as the sensor calibration curve. The best fit parameters for the slope, y-intercept, and regression coefficient are listed in the inset.

The sensor responses of the V₂O₅ coated QCM in liquid media show a very good response characteristics. When the sensor is exposed to the analyte in pure water, the sensor response reaches its maximum response level in seconds (t₉₅< 3s), and when the pure water is purged through the measurement cell, the sensor response reaches quickly the baseline level. The calculation of the response and recovery times, defined as the time needed to reach 95% of the equilibrium response or to recover a response value lower than 5% of the equilibrium response, is illustrated in figure 7. The calculated sensitivity is 12 Hz/ppm (ppm: parts per million) and the lower limit of detection (3 times the baseline noise) is 80 ppb (ppb: parts per billion) for chloroamine T. The limit of detection is 50 times lower than the value proposed by EPA for water analysis (4 ppm). The sensitivity and limit of detection values for selected amines are given in Table 1.

**Table 1: The sensitivity and limit of detection values for selected amines.**

| **Analyte** | **Sensitivity (Hz/ppm)** | **Limit of Detection (ppm)** |
|---|---|---|
| **Triethylamine** | 1.35 | 0.7 |
| **Buthylamine** | 1.77 | 0.6 |
| **Hexylamine** | 1.92 | 0.5 |

In later applications, the sensor signal at the time when the signal reaches 95% of the equilibrium signal can be used as sensor response to reduce the time necessary for a measurement. Thereby, the time necessary for the sensor to recover by purging with pure water is also reduced. The calibration curve obtained using defined test samples with different concentrations is used to determine the concentration of analyte solution of unknown concentration.

The developed sensor shows fast responses with high sensitivity to concentrations below the dangerous levels in aqueous media as defined by EPA, and high selectivity for amines and amine derivatives. The sensor can detect the amines directly in the aqueous phase without any evaporation or derivatization processes. Moreover, no responses of the newly developed sensor were observed to pesticides or other interferences without amine functional group. In this aspect, the developed sensor can be evaluated as highly selective. The developed sensor can be used for repeated tests for weeks without any observed changes in performance. Repeatability and reproducibility was investigated. The responses of the sensor were observed to be identical both in repeated sensing tests and in tests with newly coated sensors in response to the same analyte. The results show the high repeatability and successful reproduction of the sensor.

### Sensor preparation procedure:

To determine the thickness of the prepared V₂O₅ thin films, V₂O₅ was coated on a glass substrate by thermal evaporation technique under reduced pressure (9*10⁻⁶ mbar) and measured using a profilometer. As a preliminary step before the actual sensor test, potential influences of the film thickness of the sensitive materials coated on the sensor surface on the sensor performance have to be investigated. Sensors coated with films of different thickness were tested in their responses to the analytes and the optimal film thickness showing the lowest noise level of the sensor signal was determined.

The coating thickness was optimized with the help of a profilometer and the conditions for a thickness of 20 nm were determined. After the optimization process, a thin film was coated on the active metal electrode of a 5 MHz QCM with a shadow mask following the same procedure.

### Analyte preparation:

According to the solubility values in water, 0.5 ml or 20 mg analyte was added to 500 ml of pure water via a micropipette or precision balance and then mixed until a homogeneous solution has been obtained. New stock solutions were prepared before every new measurement. The desired concentration of the analytes in the test samples were obtained by diluting the stock solutions. Measured concentration levels are between 2 ppm to 740 ppm.

### Measurement System:

Sensor tests were realized using a QCM-Z500 system (KSV Instrument, Finland) with a thermostated measurement cell. The KSV QCM-Z500 determines the resonance frequency and resonance quality factor (Q) of a quartz crystal in a frequency range of 5-55 MHz in liquid media by impedance analysis.

The fundamental frequency was used as the sensor signal. In all measurements, the temperature of measurement cell was fixed to 20 °C. The temperature of the carrier liquid (pure water) and the sample liquid containing the analyte were balanced to the same temperature before the measurement.

The typical measurement protocol using the KSV QCM Z500 measurement system consist of three steps: (1) carrier liquid (pure water) is sent to the measurement cell with the QCM sensor to get the baseline level of the sensor signal, (2) analyte solution is directed into the measurement cell until the sensor signal reaches a stable equilibrium signal, and (3) again carrier liquid (pure water) is sent to the measurement cell to recover the sensor baseline. The sensor response as a frequency shift is calculated as the difference in frequency between the signal at equilibrium when analyte solution was purged through to the measurement system and the baseline level. This frequency shift is proportional to the amount of molecules on the surface of the crystal, caused by the interaction between analytes molecules and the sensitive material on the QCM. In the measurements, all other measurement parameters (density, pH etc. of solution) are kept constant.

### Sensor tests

To assess the performance of the sensors in detail the negative frequency shift as the sensor response was determined for different concentration levels of all the analytes. The responses of the sensor to the different concentration levels were approximated by a best-fit line, i.e. the so-called calibration curve. The resulting slope of the calibration curve provides the sensitivity of the sensor.

### Brief description of the figures

Figure 1 - Front view of the quartz crystal microbalance transducer (**1**) with active sensor surface (**2**).
Figure 2 - Rear view of the quartz crystal microbalance transducer (**1**) with counter electrode (**3**).
Figure 3 - Section view of quartz crystal microbalance transducer (**1**) coated with a V₂O₅ thin film **(**4) on the active sensor surface (**2**).
Figure 4 - Sketched view of a quartz crystal microbalance sensor coated with a V₂O₅ thin film (**5**) mounted in a holder (**6**) in the measurement cell (**7**) and immersed in the liquid medium (**8**).
Figure 5 - Graph of the sensor responses of a V₂O₅ coated QCM sensor as a function of measurement time when immersed in pure water and aqueous samples containing chloramine T in the concentration range of 2-40 ppm. f₀: initial frequency value of the sensor (baseline) immersed in the analyte free carrier liquid, fₛ: the frequency value when the sensor is exposed to the analyte liquid, Δf: frequency shift
Figure 6 - Graph of the sensor responses of a V₂O₅ coated QCM sensor as a function of analyte concentration (chloramine T in the concentration range 2-40 ppm), the sensitivity is defined as the slope of the analytic calibration curve showing the sensor responses to increasing analyte concentration values.
Figure 7 - Sensor response of the V₂O₅ coated QCM sensor obtained against chloramine T, indicating response time of the sensor, t_{95,c}, and recovery time, t_{95,g}.

### Numbered elements used in the figures

1. Quartz crystal microbalance
2. Active sensor surface
3. Counter electrode
4. V₂O₅ thin film
5. QCM coated with a V₂O₅ thin film (comprising **1**, **2**, **3**, and **4**)
6. Sensor holder
7. Liquid measurement cell
8. Sample liquid

### References:

1. PINHEIRO, H.M., TOURAUD, E., and THOMAS O., Dyes Pigm., 61, 121-139 (2004)
2. SNYDERWINE, E.G., SINHA, R., FELTON, J.S., and FERGUSON, L.R., Mutat. Res., 506-507, 1-8 (2002)
3. National Recommended Water Quality Criteria: 2002, EPA-822-R-02-012, United States Environmental Protection Agency, 2002
4. ONAL A., Food Chem., 103, 1475-1486 (2007)
5. Zhang, C., and Suslick, K.S., J. Am. Chem. Soc., 127, 11548-11549 (2005)
6. SAUERBREY, G., Z. Phys, 155, 206-222 (1959)
7. KING, W.H., Jr., Anal. Chem., 36, 1735-1739 (1964)
8. SPEIGHT, R.E., and COOPER, M.A., J. Mol. Recognit., 25, 451-473 (2012)
9. SHONS, A., DORMAN, F., and NAJARIAN, J., J. Biomed. Mater. Res., 6, 565-570 (1972)
10. NOMURA, T., and MINEMURA, A., NIPPON K.K., 10, 1621-1625 (1980)
11. KONASH, P.L., and BASTIAANS, G.J., Anal. Chem., 52, 1929-1931 (1980)
12. THOMPSON, M., ARTHUR, C.L., and DHALIWAL, G.K., Anal.Chem., 58, 1206-1209 (1986)
13. KANAZAWA, K.K., JOSEPH, G., and GORDON, I., Anal. Chem., 57, 1771-1772 (1985)
14. MARTIN, S.J., GRANSTAFF, V.E., and FRYE, G.C., Anal. Chem., 63, 2272-2281 (1991)
15. OKAHATA, Y., EBATO, H., and TAGUCHI, K., J. Chem. Soc., Chem. Commun., 1363-1365 (1987)
16. OKAHATA, Y., EBATO, H., and YE, X., J. Chem. Soc., Chem.Commun, 1037-1041 (1988)
17. PATEL, R., ZHOU, R., ZINSZER, K., and JOSSE, F., Anal. Chem., 72, 4888-4902 (2000)
18. MENON, A., ZHOU, R., and JOSSE, F., IEEE Trans. Ultrason. Ferroelectr. Freq. Control, 45, 1416-1418 (1998)
19. PEJCIC, B., BARTON, C., CROOKE, E., EADINGTON, P., JEE, E., and ROSS, A., Sens. Actuators, B, 135, 436-443 (2009)
20. ZHOU, R., HAIMBODI, M., EVERHART, D., and JOSSE, F., Sens. Actuators, B, 35-36, 176-182 (1996)
21. ROSLER, S., LUCKLUM, R., BORNGRÄBER, R., HARTMANN, J., and HAUPTMANN, P., Sens. Actuators, B, 48, 415-424 (1998)
22. LUCKLUM, R., ROSLER, S., HARTMANN, J., and HAUPTMANN, P., Sens. Actuators, B, 35-36, 103-111 (1996)
23. SARTORE, L., BARBAGLIO, M., BORGESE, L., BONTEMPI, E., Sens. Actuators, B, 155, 538-544 (2011)
24. CYGAN, M.T., COLLINS, G.E., DUNBAR, T.D., ALLARA, D.L., GIBBS, C.G., and GUTSCHE, C.D., Anal. Chem., 71, 142-148 (1999)
25. ZHOU, X.C., NG, S.C., CHAN, H.S.O., and LI, S.F.Y., Sens. Actuators, B, 42, 137-144 (1997)
26. ERBAHAR, D.D., HARBECK, M., GÜMÜ 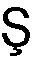 , G., GÜROL, I., and AHSEN, V., Sens. Actuators, B, 190C, 651-656 (2014)
27. HARBECK, M., ERBAHAR, D.D., GÜROL, I., MUSLUOǦLU, E., AHSEN, V., and ÖZTÜRK, Z.Z., Sens. Actuators, B, 150, 346-354 (2010)
28. HARBECK, M., ERBAHAR, D.D., GÜROL, I., MUSLUOǦLU, E., AHSEN, V., and ÖZTÜRK, Z.Z., Sens. Actuators, B, 155, 298-303 (2011)
29. ERBAHAR, D.D., GUROL, I., AHSEN, V., ÖZTÜRK, Z.Z., MUSLUOǦLU, E., and HARBECK, M., Sens. Lett., 9, 745-748 (2011)
30. ERBAHAR, D.D., GÜROL, I., GÜMÜ 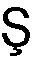 , G., MUSLUOǦLU, E., ÖZTÜRK, Z.Z., AHSEN, V., and HARBECK, M., Sens. Actuators, B, 173C, 562-568 (2012)
31. ERBAHAR, D.D., HARBECK, M., GÜROL, I., GÜMÜ 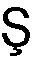 , G., MUSLUOǦLU, E., ÖZTÜRK, Z.Z., and AHSEN, V., J. Porphyrins Phthalocyanines, 17, 989-995 (2013)
32. PEJCIC, B., MYERS, M., RANWALA, N., BOYD, L., BAKER, M., and ROSS, A., Talanta, 85, 1648-1657 (2011)
33. PEJCIC, B., CROOKE, E. BOYD, L., DOHERTY, C.M., HILL, A.J., MYERS, M., and WHITE, C., Anal. Chem., 84, 8564-8570 (2012)
34. HAUPT, K., NOWORYTA, K., and KUTNER, W., Anal. Commun., 36, 391-393 (1999)
35. MALITESTA, C., LOSITO, I., and ZAMBONIN, P.G., Anal.Chem., 71, 1366-1370 (1999)
36. DICKERT, F.L., TORTSCHANOFF, M., BULST, W.E., and FISCHERAUER, G., Anal. Chem., 71, 4559-4563 (1999)
37. PERCIVAL, C.J., STANLEY, S., GALLE, M., BRAITHWAITE, A., NEWTON, M.I., McHALE, G., and HAYES, W., Anal. Chem., 73, 4225-4228 (2001)
38. QUY, N.V., HUNG, T.M., THONG, T.Q., TUAN, L.A., HUY, T.Q., HOA, N.D., Curr. Appl Phys., 13, 1581-1588 (2013)
39. YANG, M., and HE, J., J. Colloid Interface Sci., 368, 41-48 (2012)
40. YANG, M., HE, J., HU, X., and YAN, C., Sens. Actuators, B, 155, 692-698 (2011)
41. YANG, M., HE, J., HU, X., YAN, C., and CHENG, Z., Environ. Sci. Technol., 45, 6088-6094 (2011)
42. ZHAO, Y., HE, J., YANG, M., GAO, S., ZUO, G., YAN, C., and CHENG, Z., Anal. Chim. Acta, 654, 120-126 (2009)
43. ZHAO, Y., CHEN, H., WANG, X., HE, J., YU, Y., and HE, H., Anal. Chim. Acta, 675 (2010) 36-41
44. ZHAO, Y., DU, X., WANG, X., HE, J., YU, Y., and HE, H., Sens. Actuators, B, 151, 205-211 (2010)
45. LU et al., US 8,377,683 B2 Patent (2013)
46. YAMAZOE, N., SAKAI, G., and SHIMANOE, K., Catal. Surv. Asia, 7, 63-75 (2003)
47. RAIBLE, I., BURGHARD, M., SCHLECHT, U., YASUDA, A., and VOSSMEYER, T., Sens. Actuators, B, 106, 730-735 (2005)
48. BESNARD, I., et al., US 2009/0084162 A1 Patent (2009)
49. BESNARD, I., et al., WO 03/046536 A1 Patent (2002)
50. LU, J.G., CHANG, P., and FAN, Z., Mater. Sci. and Eng. R, 52, 49-91 (2006)
51. YIN, X., FAHMI, A., HAN, H., ENDOU, A., AMMAL, C.S.S., KUBO, M., TERAISHI, K., and MIYAMOTO, A., J. Phys. Chem. B, 103, 3218-3224 (1999)

## Claims

1. A use of a chemical sensor (5) for detecting analytes, said sensor having a piezoelectric substrate (1), two metal electrodes on the substrate (2-3), and a metal oxide sensitive layer (4) on the substrate, wherein the metal oxide is vanadium pentoxide (V2O5), **characterized in that** the sensor is directly immersed in liquid medium having the analytes

2. The use of a chemical sensor according to claim 1 wherein the substrate is a quartz crystal.

3. The use of a chemical sensor according to claim 1 wherein the metal electrodes (2-3) on the substrate are gold electrodes.

4. The use of a chemical sensor according to claim 1 wherein the analytes in the liquid medium that are being detected are amines and amine derivatives.

5. The use of a chemical sensor according to claim 4 wherein the amine and amine derivatives are at least one of the compounds butylamine, triethylamine, hexylamine, chloramine T.

## Patentansprüche

1. Verwendung eines chemischen Sensors (5) zum Erfassen von Analyten, welcher Sensor ein piezoelektrisches Substrat (1), zwei Metallelektroden auf dem Substrat (2-3) und eine metalloxidempfindliche Schicht (4) auf dem Substrat aufweist, wobei das Metalloxid Vanadiumpentoxid (V2O5) ist, **dadurch gekennzeichnet, dass** der Sensor direkt in ein flüssiges Medium mit den Analyten eingetaucht ist.

2. Verwendung eines chemischen Sensors nach Anspruch 1, wobei das Substrat ein Quarzkristall ist.

3. Verwendung eines chemischen Sensors nach Anspruch 1, wobei die Metallelektroden (2-3) auf dem Substrat Goldelektroden sind.

4. Verwendung eines chemischen Sensors nach Anspruch 1, wobei die Analyten in dem flüssigen Medium, die nachgewiesen werden, Amine und Aminderivate sind.

5. Verwendung eines chemischen Sensors nach Anspruch 4, wobei das Amin und die Aminderivate mindestens eine der Verbindungen Butylamin, Triethylamin, Hexylamin, Chloramin T sind.

## Revendications

1. Utilisation d'un capteur chimique (5) pour détecter des analytes, ledit capteur ayant un substrat piézoélectrique (1), deux électrodes métalliques sur le substrat (2-3), et une couche sensible à l'oxyde métallique (4) sur le substrat dans laquelle l'oxyde métallique est du pentoxyde de vanadium (V2O5), **caractérisée en ce que** le capteur est directement immergé dans un milieu liquide ayant les analytes.

2. Utilisation d'un capteur chimique selon la revendication 1 dans laquelle le substrat est un cristal de quartz.

3. Utilisation d'un capteur chimique selon la revendication 1 dans laquelle les électrodes métalliques (2-3) sur le substrat sont des électrodes en or.

4. Utilisation d'un capteur chimique selon la revendication 1 dans laquelle les analytes dans le milieu liquide qui sont détectés sont des amines et des dérivés aminés.

5. Utilisation d'un capteur chimique selon la revendication 4 dans laquelle l'amine et les dérivés d'amine sont au moins l'un des composés butylamine, triéthylamine, hexylamine, chloramine T.
